# EUROPEAN PATENT SPECIFICATION

(11) **EP 4 384 496 B1**
(45) Date of publication and mention of the grant of the patent: **24.09.2025**
(21) Application number: 22761628.1
(22) Date of filing: 12.08.2022
(51) Int. Cl.: C07C 269/04, C07C 271/04, C07C 303/40, C07C 311/58, C07D 209/52

(54) **FLOW SYNTHESIS PROCESS FOR THE PRODUCTION OF SULFONYLUREA COMPOUNDS**
FLUSSSYNTHESEVERFAHREN ZUR HERSTELLUNG VON SULFONYLHARNSTOFFVERBINDUNGEN
PROCÉDÉ DE SYNTHÈSE EN FLUX POUR LA PRODUCTION DE COMPOSÉS DE SULFONYLURÉE

(30) Priority: 13.08.2021 GB 202111677
(43) Date of publication of application: 19.06.2024
(73) Proprietor: Nelson Mandela University, 6001 Port Elizabeth (ZA)
(72) Inventor: WATTS, Paul, 6001 Port Elizabeth (ZA); SAGANDIRA, Cloudius Ray, 6001 Port Elizabeth (ZA)
(74) Representative: Abel & Imray LLP
(86) International application number: PCT/IB2022/057538
(87) International publication number: WO 2023/017474

(56) References cited:
- G.V. AMBULGEKAR, ET AL.: "A novel and facile process for the synthesis of gliclazide", LETTERS IN ORGANIC CHEMISTRY, vol. 15, no. 9, September 2018 (2018-09-01), Bentham Science Publishers, Sharjah, UAE, pages 760 - 765, XP009540527, ISSN: 1570-1786, DOI: 10.2174/1570178615666180102161540
- C.R. SAGANDIRA, ET AL.: "Rapid multigram-scale end-to-end continuous-flow synthesis of sulfonylurea antidiabetes drugs: gliclazide, chlorpropamide and tolbutamide", SYNTHESIS, vol. 54, no. 05, 22 November 2021 (2021-11-22), Georg Thieme Verlag, Stuttgart, DE, pages 1365 - 1374, XP055982679, ISSN: 0039-7881, DOI: 10.1055/a-1664-2282

## Description

### INTRODUCTION

This invention relates to a process for producing sulfonylurea compounds, including gliclazide, chlorpropamide and tolbutamide, and pharmaceutically acceptable salts thereof, in particular to a flow synthesis process for producing these compounds.

### BACKGROUND

Sulfonylureas, discovered by Janbon and co-workers in 1942, have been extensively used for treatment of type 2 diabetes for nearly 50 years. Despite several anti-diabetic agents on the market, sulfonylureas remain one of the most prescribed due to affordability, possibility of mono-dosing and presence of an association with metformin in the same tablet.

Diabetes mellitus is an endocrinological disorder that causes high blood sugar. According to the International Diabetes Federation (IDF) 425 million people had diabetes in 2017 worldwide with 1.8 million cases being reported in South Africa. Furthermore, it is predicted that about 552 million people worldwide will have diabetes by 2030.

Gliclazide **1a,** chlorpropamide **1b** and tolbutamide **1c** are some of the sulfonylurea drugs used for the treatment of diabetes mellitus. Although there are various synthetic approaches in literature towards these drugs, these processes are all inefficient.

Further, existing synthesis methodologies for the production of these compounds have essentially been based on standard stirred batch reactor type processes, wherein significant volumes of organic solvents are used.

Micro reactor technology (MRT), more recently branded 'flow chemistry', is an emerging technique that enables those working in research and development to rapidly screen reactions utilising continuous flow, leading to the identification of reaction conditions that are suitable for use at a production level. Furthermore, in addition to using conventional reaction methodology, the inherent safety associated with the use of small reactor volumes enables users to employ reaction conditions previously thought to be too hazardous for use within a production environment; such as extreme reaction conditions or the use/generation of 'hazardous' compounds. Consequently, the type of reactions available to the chemist increases through the use of this technology.

Although it is known to be desirable to transfer batch processes to flow synthesis, it often requires significant reaction condition and reagent modifications which are not necessarily obvious, considering the vast amount of permutations in reagents and reaction conditions and the unpredictable nature of chemistry in general. Furthermore, flow synthesis methodology has inherent challenges that has to be overcome, in particular for continuous multistep reactions.

The present invention seeks to address some of the shortcomings of the prior art by providing new flow chemistry processes for producing sulfonylurea compounds, including gliclazide, chlorpropamide and tolbutamide.

The prior art discloses a process for the synthesis of glicazide in which an alkyl or aryl chloroformate derivative of hexahydrocyclopenta[c]pyrrol-2-amine is formed and then reacted with p- toluenesulfonamide in the presence of a base (G.V. Ambulgekar, et al., Letters in Organic Chemistry, 2018, 15(9), 760-765*).* The reactions in the prior art are done as a batch-wise process and the prior art document does not mention the possibility of carrying them out as a flow synthesis process.

### SUMMARY OF THE INVENTION

According to a first aspect to the present invention there is provided a flow synthesis process for producing a sulfonylurea compound of the Formula **1** or its pharmaceutically acceptable salts, 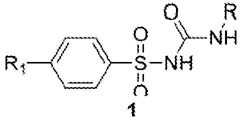 the process comprising the steps of:
a) preparing the carbamate of Formula **2** 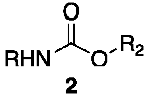 by reacting free amine RNH₂ or amine HCl salt with haloformate of Formula **4** in the presence of a base selected from 1,8-diazabicyclo[5.4.0]undec-7-ene (DBU), trimethylamine (TEA), and tributyl amine (TBA), wherein the free amine RNH2 or amine HCl salt to base molar ratio is about 1:1.5 to about 1:3, 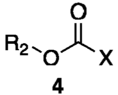
b) reacting the carbamate of Formula 2 with the sulfonamide of Formula **3** in the presence of a base selected from 1,8-diazabicyclo[5.4.0]undec-7-ene (DBU), trimethylamine (TEA), and tributyl amine (TBA), 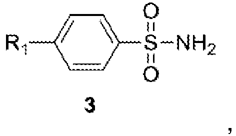 wherein
   R₂ is selected from aryl and alkyl,
   R₁ is selected from Cl and -CH₃,
   R is selected from propyl, butyl, and 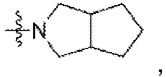 and
   X is selected from Cl, Br, and I.

In one embodiment, X is Cl.

In one embodiment, the process is a continuous multi-step process without the isolation of any intermediates.

Preferably, the reaction of step (a) is performed using aryl chloroformate. More preferably, the reaction of step (a) is performed using phenyl chloroformate.

In one embodiment, the reaction of step (a) is performed using aryl iodoformate or aryl bromoformate, in particular phenyl iodoformate phenyl bromoformate. Preferably, the reaction of step (a) is performed in an organic solvent selected from the group consisting of chloroform, dichloromethane, acetonitrile, and mixtures thereof.

More preferably, the reaction of step (a) is performed in chloroform.

In one embodiment, the base used in step (a) and step (b) is the same. Preferably, the reaction of step (a) is thermally controlled to a temperature equal to about room temperature or less.

More preferably, the reaction of step (a) is thermally controlled to a temperature between about 0 °C and about room temperature.

In a preferred embodiment, the free amine RNH₂ or amine HCl salt has a concentration of about 0.1M to about 2M.

In a preferred embodiment, the sulfonamide has a concentration of about 0.1M to about 2M.

In a preferred embodiment, in step (b) the sulfonamide to carbamate molar ratio is about 1:1 to about 1:2.

In a particularly preferred embodiment, in step (b) the sulfonamide to carbamate molar ratio is about 1:1.5.

In a preferred embodiment, in step (b) the sulfonamide to base molar ratio is about 1:2 to about 1:5.

Preferably, the reaction of step (b) is thermally controlled to a temperature between about 70 °C and about 100 °C.

More preferably, the reaction of step (b) is thermally controlled to a temperature of about 80 °C.

Preferably, the reaction of step (b) is performed in an organic solvent selected from the group consisting of chloroform, dichloromethane, acetonitrile, and mixtures thereof.

More preferably, the reaction of step (b) is performed in acetonitrile.

### BRIEF DESCRIPTION OF THE DRAWINGS

The invention will now be described in more detail with reference to the following non-limiting embodiments and figures in which:
- **Figure 1**: shows the effect of the molar equivalent ratio of base (DBU) and carbamate **2a** to sulfonamide **3a** in the reaction (step (b)) towards gliclazide **1a**;
- **Figure 2**: shows the effect of variations in temperature, molar equivalents, and residence time of the reaction of step (b) towards gliclazide **1a**; and
- **Figure 3**: shows the effect of variations in molar equivalents and residence time of the reaction of step (b) towards chlorpropamide **1b** and tolbutamide **1c**.

### DETAILED DESCRIPTION OF A PREFERRED EMBODIMENT

The present invention will now be described more fully hereinafter with reference to the accompanying figures, in which some of the non-limiting embodiments of the invention are shown.

The invention as described hereinafter should not be construed to be limited to the specific embodiments disclosed, with slight modifications and other embodiments intended to be included within the scope of the invention.

Although specific terms are employed herein, they are used in a generic and descriptive sense only and not for purposes of limitation.

As used herein, throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having", "including", and variations thereof used herein, are meant to encompass the items listed thereafter, and equivalents thereof as well as additional items.

The present invention provides for a flow synthesis process for producing sulfonylurea compounds and pharmaceutically acceptable salts thereof, in particular to a flow synthesis process for producing gliclazide, chlorpropamide and tolbutamide. In a particularly preferred embodiment, the invention provides for a continuous multistep flow synthesis process for producing gliclazide, chlorpropamide and tolbutamide without the isolation of any intermediates.

Although it is known to be desirable to transfer exiting batch processes or methodologies to flow synthesis systems, it requires significant reaction condition and reagent type modifications which are not obvious to the skilled person, in particular considering the endless permutations in reagents and reaction conditions available. Furthermore, flow synthesis has inherent challenges that has to be overcome, in particular for continuous multistep reactions. The inventors of the present invention overcame these challenges, and developed highly efficient and rapid methods for the synthesis of various sulfonylurea compounds, as disclosed herein.

As shown in Scheme 1 below, the inventors have envisioned the preparation of sulfonylurea **1** (Gliclazide **1a,** chlorpropamide **1b** and tolbutamide **1c**) from the reaction of the key carbamate **2** intermediate with sulfonamide **3.** Whilst sulfonamide **3** is commercially available, carbamate **2** is not readily available. Carbamate **2** was prepared from the treatment of free amine **5** or amine HCl salt with chloroformate **4.**

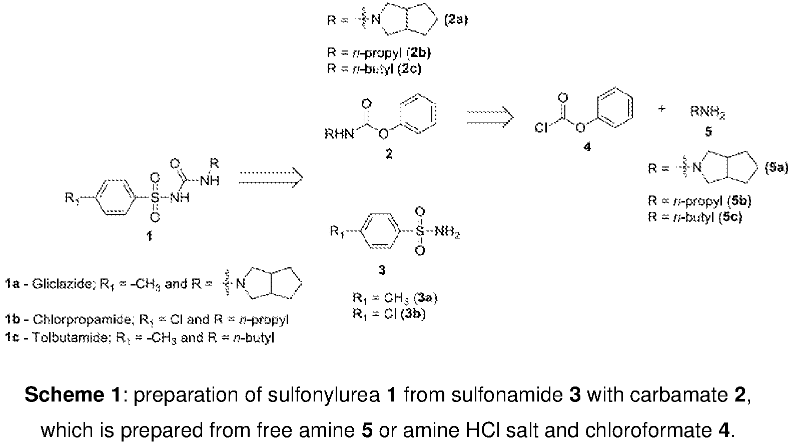

### General Experimental Procedures

Chemicals were supplied by Sigma Aldrich, Merck and Industrial Analytical and were used as received. Anhydrous solvents were supplied by Sigma Aldrich, and maintained by drying over appropriately activated molecular sieves during use.

Column chromatography was performed using Fluka Chemie silica gel 60 as the stationary phase, and mixtures of ethyl acetate and hexane of varying polarity were used as the mobile phase. Unless otherwise stated, thin layer chromatography (TLC) was done using Merck Kieselgel 60 HF254 aluminium backed TLC plates with mixtures of ethyl acetate and hexane of varying polarity as eluent. TLC visualisation was done by fluorescence on exposure to short wave ultra violet (UV) light (λ 254 nm) in a Camag UV cabinet.

Nuclear magnetic resonance (NMR) spectra were recorded at room temperature as solutions in deuterated chloroform (CDCl₃) or deuterated dimethyl sulfoxide (DMSO-d₆). A Bruker Avance-400 spectrometer (400 MHz) was used to record the spectra and the chemical shifts are reported in parts per million (ppm) with coupling constants in Hertz (Hz).

Infra-red spectra were recorded from 4000 to 500 cm⁻¹ using a Bruker spectrometer and peaks (Vₘₐₓ) reported in wavenumbers (cm⁻¹). Melting points of all compounds were determined using a Staurt Melting Point Apparatus SMP30.

High performance liquid chromatography (HPLC) data was obtained using Agilent 1220 with a UV/Vis detector: HPLC analysis was performed on ACE Generix 5 C18(2) column (150 mm x 4.6 mm i.d.) at ambient temperature using an isocratic system. The mobile phase consisted of 70 % acetonitrile and 30 % water. The sample injection volume was 1 µl, eluted at a flow rate of 1.5 ml/min and detected at 230 nm with a run time of 7 min.

The collected carbamate solutions were washed with water and extracted into DCM. The products were concentrated at room temperature under reduced pressure to afford a solid. For spectroscopic analysis, the products were washed with acetone and air dried. The products were characterized by FTIR and NMR.

The collected sulfonylurea solutions were concentrated under reduced pressure and redissolved in DCM and washed with water. The organic layer was concentrated under vacuum to afford a white solid. The products were purified with methanol and oven dried at 60 °C and characterized by FTIR and NMR.

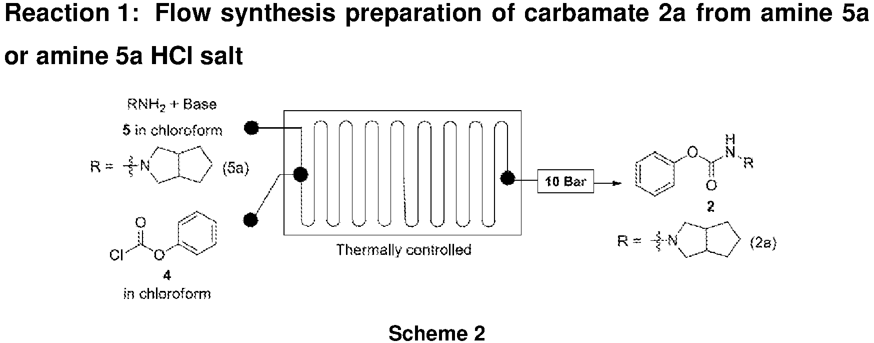

In one embodiment of the invention, as shown in Scheme 2 above, amine 5a was treated with chloroformate **4** in the presence of a base in a continuous flow system to afford the desired carbamate **2.** The effect of residence time and temperature on the reaction was investigated in flow. The results of the investigations are presented in Table 1 below.

**Table 1: The investigation of residence time and temperature in the reaction towards carbamate.**

| Entry | Res. Time (s) | Temp. (°C) | Conversion (%)^{e} |
|---|---|---|---|
| 1 (a) | 15 | 0 | 78 |
| 2 (a) | 15 | r.t | 79 |
| 3 (a) | 30 | r.t | 100 |
| 4 (a) | 15 | 100 | 72 |
| 5 (a) | 60 | 100 | 59 |
| 6 (b) | 30 | r.t | 54 |
| 7 (d) | 30 | r.t | 78 |
| 8 (d) | 30 | 0 | 100 (96)^{f} |

| | | | |
|---|---|---|---|
| Standard conditions: ^{a}Amine **5a** (0.1 M, 1 Equiv.), DBU or TEA (1 Equiv.) and chloroformate **4** (1 Equiv.), ^{b}Amine **5a** (0.1 M, 1 Equiv.), chloroformate 4 (1 Equiv.), ^{d}Amine **5a** (1 M, 1 Equiv.), DBU or TEA (1 Equiv.), chloroformate **4**, ^{e}Conversion determined by HPLC, ^{f}Number in parentheses corresponds to isolated yield. | | | |

Carbamate **2a** was formed in 78 % conversion at 0 °C in 15 s (Table 1, Entry 1). Increasing temperature to room temperature had little effect on the conversion (Table 1, Entry 2). However, an increase in temperature to 100 °C was accompanied by a decrease in conversion (Table 1, Entry 4 and 5). Further decrease in conversion was observed with increase in residence time (Table 1, Entry 5). This can be attributed to the thermal decomposition of carbamate **2a** as confirmed by the formation of phenol. Performing the reaction at room temperature and 30 s residence time using 0.1 M solutions afforded full conversion (Table 1, Entry 3). However, the use of concentrated solutions (1 M) at room temperature was accompanied by a decrease in conversion (Table 1, Entry 7).

The carbamate formation reaction is exothermic and its exothermicity is more pronounced with increased reaction concentration. Since carbamate **2a** is thermally unstable, it decomposes from the heat of reaction if the reaction temperature is not properly controlled. Consequently, carbamate **2a** was prepared in full conversion and 96 % isolated yield at 0 °C and 30 s using concentrated reagents (Table 1, Entry 8). Carbamate **2a** was prepared in 54 % yield in the absence of DBU (Table 1, Entry 6).

Amine **5a** is commercially supplied as amine **5a** HCl salt. With large scale synthesis carbamate **2a** in mind, the inventors found it desirable to investigate the preparation of carbamate **2a** directly from the amine **5a** HCl salt without the need to first prepare the free amine **5a** in a batch process. Amine HCl presents challenges with dissolution, in a particular in a micro flow system sensitive to system blockages, in particular with low temperature reaction conditions being preferred. It was therefore challenging to find an appropriate organic solvent to dissolve amine **5a** HCl. Surprisingly, the inventors found that chloroform, dichloromethane and acetonitrile in the presence of equimolar base, including the organic base 1,8-Diazabicyclo[5.4.0]undec-7-ene (DBU), provided a satisfactory dissolution profile. In one example, amine **5a** HCl salt was dissolved in chloroform in the presence of DBU pumped separately with chloroformate **4** into a 2 ml glass reactor to investigate the preparation of carbamate **2a** (Scheme 2 and Table 2).

**Table 2: The investigation of residence time and temperature in the reaction towards carbamate 2a from amine 5a HCl salt**

| Entry | Res. Time (s) | Temp. (°C) | Conversion (%)^{d} |
|---|---|---|---|
| 1 (a) | 30 | 0 | 49 |
| 2 (a) | 60 | 0 | 52 |
| 3 (b) | 30 | 0 | 97(93)^{e} |
| 4 (b) | 60 | 0 | 100 |
| 5 (c) | 60 | 0 | 76 |

| | | | |
|---|---|---|---|
| Standard conditions: ^{a}Amine **5a** HCl (1 M, 1 Equiv.), DBU (1 Equiv.) and chloroformate 4 (1 Equiv.), ^{b}Amine **5a** HCl (1 M, 1 Equiv.), DBU (2 Equiv.), chloroformate **4** (1 Equiv.), ^{c}Amine **5a** HCl (1 M, 1 Equiv.), DBU (3 Equiv.), chloroformate **4** (1 Equiv.), ^{d}Conversion determined by HPLC, ^{e}Number in parentheses corresponds to isolated yield. | | | |

The preparation of carbamate **2a** from amine **5a** HCl in the presence of equimolar DBU was afforded in 49 - 52 % conversion (Table 2, Entry 1 and 2). However, a conversion of 97 % and 93 % isolated yield was afforded in the presence of DBU (2 Equiv.) and full conversion was afforded in 60 s (Table 2, Entry 3 and 4). DBU (2 Equiv.) was found to be desirable to keep the free amine **5a** in solution in chloroform as well as mopping the HCl formed in the reaction. The use of DBU (3 Equiv.) resulted in a decrease in conversion (Table 2, Entry 5). The inventors managed to prepare carbamate **2a** at a multigram scale in a Chemtrix Kiloflow^{®} continuous flow system (24 ml total reactor volume) using the following reaction conditions Amine **5a** HCl (1 Equiv.), DBU (2 Equiv.), chloroformate (1 Equiv.), 30 s residence time and 0 °C. Carbamate **2a** was afforded in 93 % isolated yield with a throughput of 330 g/h.

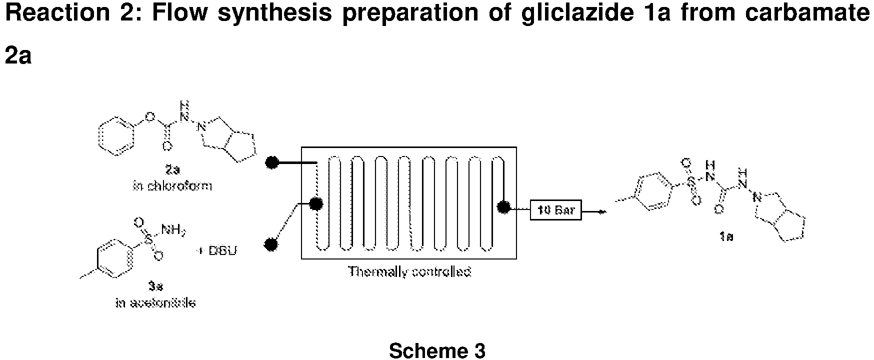

Following the successful preparation of carbamate **2a** in flow, the inventors proceeded with the preparation of gliclazide **1a** from carbamate **2a** in flow. As is shown in Scheme 3 above, carbamate **2a** was treated with sulfonamide **3a** in the presence of a base in a 2 ml glass reactor. A summary of preliminary investigations is shown in Table 3.

**Table 3: The investigation of residence time and temperature in the reaction towards gliclazide 1a from carbamate 2a**

| Entry | Res. Time (min) | Temp. (°C) | Conversion (%)^{c} |
|---|---|---|---|
| 1 (a) | 1 | r.t | 8 |
| 2 (a) | 2 | r.t | 11 |
| 3 (a) | 2 | 80 | 46 |
| 4 (b) | 2 | 80 | 57 |
| 5 (b) | 2 | 100 | 49 |

| | | | |
|---|---|---|---|
| Standard conditions: ^{a}Sulfonamide **3a** (0.5 M, 1 Equiv.) premixed with DBU (2 Equiv.), carbamate **2a** (1Equiv.), ^{b}Sulfonamide **3a** (0.5 M, 1 Equiv.) premixed with DBU (2 Equiv.), carbamate **2a** (1.5 Equiv.), ^{c}Conversion determined by HPLC. | | | |

The treatment of carbamate **2a** with sulfonamide **3a** at room temperature and 1 min residence time afforded gliclazide **1a** in a less than desirable conversion of 8 % (Table 3, Entry 1). The conversion only increased slightly to 11% with an increase in residence time (Table 3, Entry 2). An increase in temperature to 80 °C resulted in increase in conversion to 46 % (Table 3, Entry 3), which was still not considered sufficient. The inventors attempted to increase conversion by increasing the carbamate **2a** equivalents, which improved gliclazide **1a** synthesis (Table 3, Entry 4) to 57%. Further attempts by the inventors to improve the conversion towards gliclazide **1a** included increasing the reaction temperature to 100 °C. However, this change in reaction conditions was accompanied by a decrease in conversion (Table 3, Entry 5).

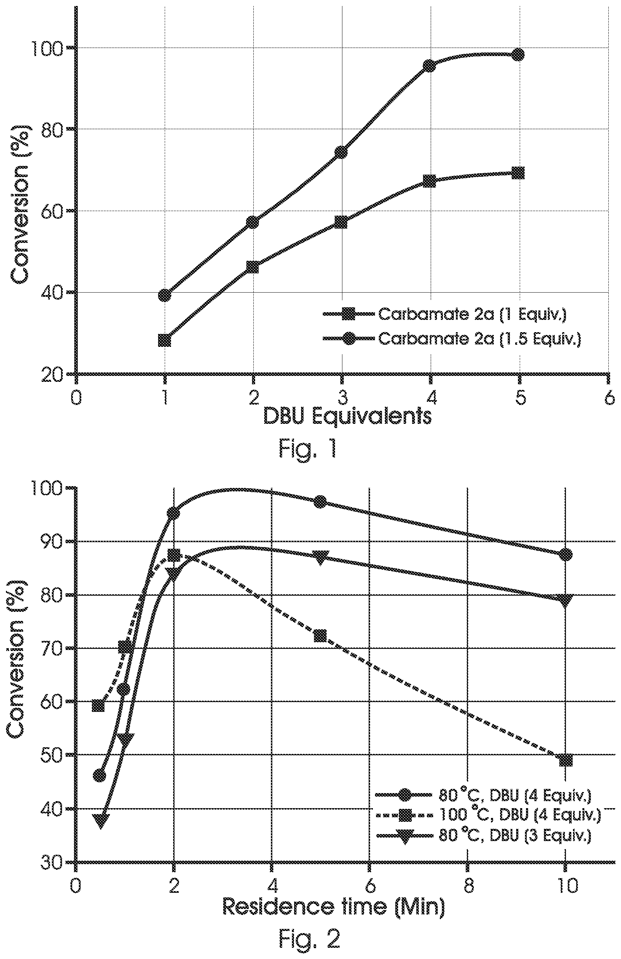

The inventors then surprisingly found that the correct molar equivalent ratio of DBU and carbamate **2a** equivalents was central in obtaining the required conversion towards gliclazide **1a.** Figure 1 shows the results for investigations on the reaction when treating sulfonamide **3a** (0.5 M) premixed with DBU with carbamate **2a** at 80 °C in a 2 ml glass reactor for 2 min residence time.

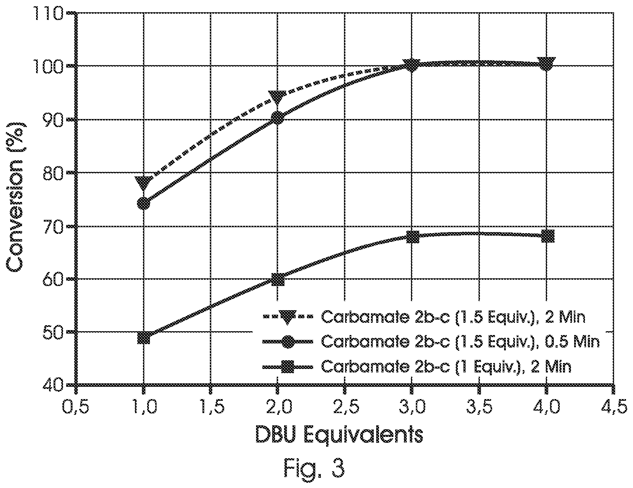

Gliclazide **1a** formation improved significantly with an increase in both carbamate **2a** and base (DBU) equivalents (see Figure 1). A conversion of 98 % was obtained in the presence of carbamate **2a** (1.5 Equiv.) and DBU (5 Equiv.), while a conversion of 95% was obtained with carbamate **2a** (1.5 Equiv.) and DBU (4 Equiv.). Figure 2 shows the results obtained in the reactions of sulfonamide **3a** (0.5 M, 1 Equiv.) with DBU (4 Equiv.) and carbamate **2a** (1.5 Equiv.), as well as sulfonamide 3a (0.5 M, 1 Equiv.) with DBU (3 Equiv.) and carbamate **2a** (1.5 Equiv.) in a 2 ml glass reactor (Figure 3 and 2).

As can be seen from Figure 2, conversion generally increased with increase in residence time, but only up to a certain point. The decrease in conversion with increase in residence time is more evident at 100 °C. This can likely be attributed to the decomposition of carbamate **2a.** In the particular flow synthesis setup used in this embodiment, the optimum conditions were found to be 80 °C and 2 min residence time using sulfonamide **3a** (0.5 M, 1 Equiv.) premixed with DBU (4 Equiv.) and carbamate (1.5 Equiv.) to afford gliclazide **1a** in and exceptional conversion of 95 %. At the determined optimum conditions for gliclazide **1a,** the reaction was scaled up in a Chemtrix Kiloflow^{®} continuous flow system (18.4 ml total reactor volume) to afford gliclazide **1a** in 93 % conversion and 91 % isolated yield with a throughput of 41 g/h.

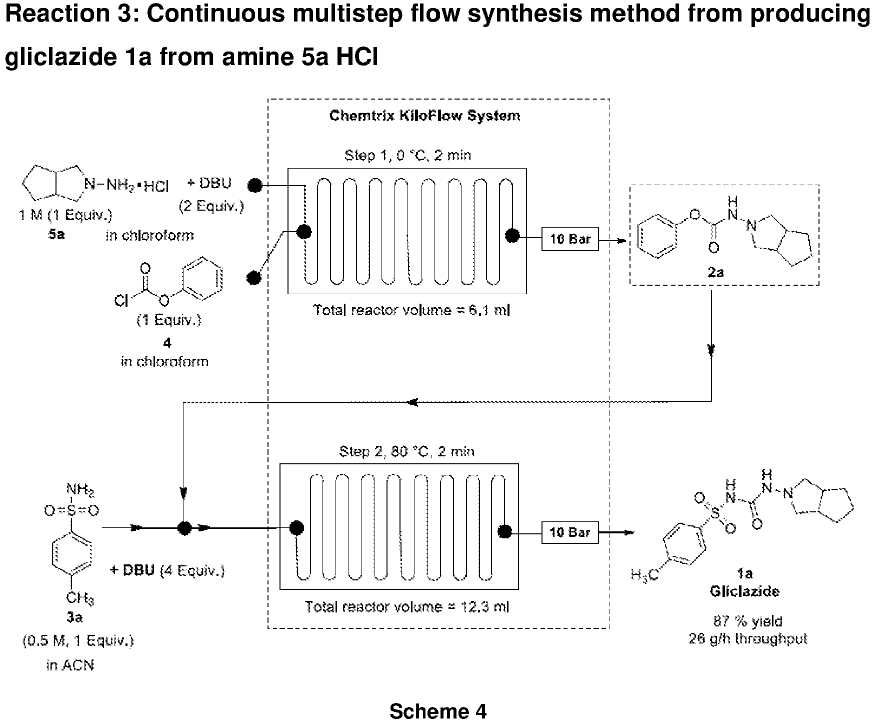

Based on the learnings from the experiments detailed above in respect of the single step reactions, the inventors successfully performed a multistep synthesis of gliclazide **1a** from amine **5a** HCl (1 M, 1 Equiv.) via the carbamate **2a** intermediate. In this embodiment of a continuous multistep flow synthesis reaction, the process was performed in a Chemtrix Kiloflow^{®} flow system (18.4 ml total reactor volume) to afford gliclazide **1a** in 90 % conversion and 87 % isolated yield with a throughput of 26 g/h (Scheme 4). It will be appreciated that the reagents and the reaction conditions shown in Scheme 4, including the use of amine **5a** HCl salt, can be altered according to the teachings in the experiments above and other substitutions which the skilled person would consider to have a high likelihood of success.

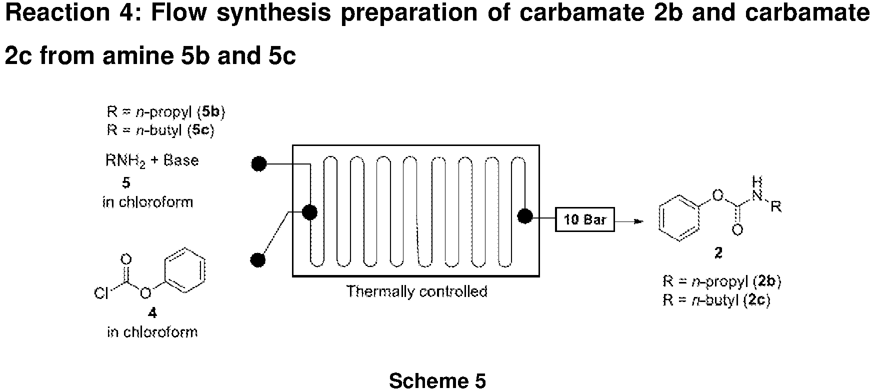

The respective carbamates **2b** and **2c** for chlorpropamide **1b** and tolbutamide **1c** were prepared starting from the conditions found to be favourable for the flow preparation of carbamate **2a** (amine 5 0.1 M (1 Equiv.), DBU (2 Equiv.), 0 °C and 0.5 min) in a 2 ml reactor (Scheme 5 and Table 4). Due to close chemical reactivities of amines **5b** and **5c,** both carbamates **2b** and **2c** were formed in same conversions at comparable reaction conditions (Table 4).

**Table 4: The preparation of carbamate 2 in flow**

| Entry | Chloroformate **4** Equivalents | Amine **5** Equivalents | Base | Carbamate **2** (%)^{b} |
|---|---|---|---|---|
| 1 | 1 | 1 | DBU (2 Equiv.) | 100 |
| 2 | 1 | 1 | DBU (1 Equiv.) | 100 |
| 3 | 1 | 1 | TBA | 100 |
| 4 | 1 | 1 | 0 | 55 |
| 5^{a} | 1 | 1 | TBA | 100 |

| | | | | |
|---|---|---|---|---|
| Standard conditions: ^{a}Amine **5b** and **5c** (2 M, 1 Equiv.), Chloroformate **4** (1 Equiv.). ^{b}Conversion determined by HPLC. | | | | |

The treatment of amine **5** (**b** and **c**) (0.1 M, 1 Equiv.) with phenyl chloroformate **4** in the presence of DBU (2 Equiv.) at 0 °C for 0.5 min afforded respective carbamates **2** (**b** and **c**) in 100 % conversion (Table 4, Entry 1). Full conversion was still afforded when DBU (1 Equiv.) (Table 4, Entry 2). The use of an alternative base tributyl amine (TBA) (1 Equiv.) afforded carbamate **2** (**b** and **c**) in full conversion (Table 4, Entry 3). A conversion of 55 % was obtained in the absence of a base as a result of the HCl formed that hinders the reaction by reacting with amine **5** (**b** and **c**) (Table 4, Entry 4). Carbamates **2** (**b** and **c**) were also prepared successfully using 2 M reagents in full conversion (Table 4, Entry 5). However, the use of concentrations above 2 M resulted in reactor clogging.

Carbamates **2b** and **2c** were prepared at multigram scale in a Chemtrix Kiloflow^{®} continuous flow system (24 ml total reactor volume) with amine **5** (**b** and **c**) (2M, 1 Equiv.), TBA (2 Equiv.), chloroformate **4** (1 Equiv.), 0.5 min residence time and 0 °C. Both carbamate **2b** and **2c** were prepared at and isolated yield of 98 % with a throughput of 516 g/h and 556 g/h respectively.

It will be appreciated by those skilled in the art that, in line with the preparation of carbamate **2a** from amine **5a** HCl salt, carbamates **2b** and **2c** could similarly be prepared in flow from the HCl salts of amine **5b** and amine **5c**.

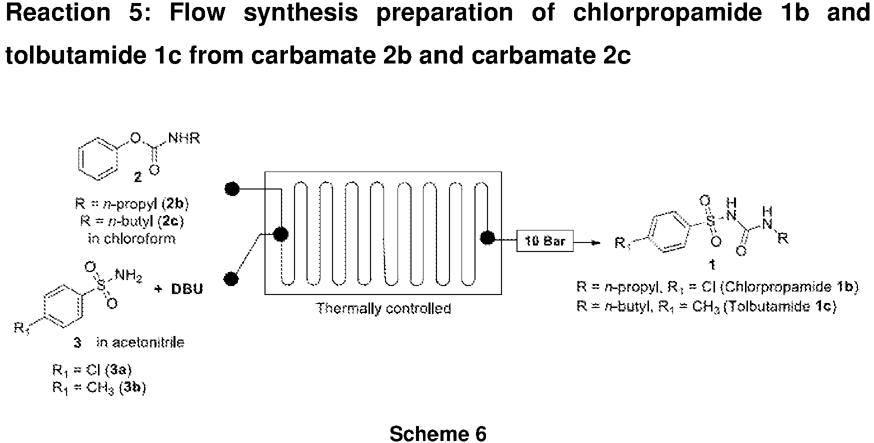

Chlorpropamide **1b** and tolbutamide **1c** were prepared from carbamates 2b and **2c** respectively in a 2 ml glass reactor at the conditions found to be favourable for gliclazide **1a** synthesis from carbamate **2a,** i.e. (Sulfonamide **3a** (1 M, 1 Equiv.) premixed with DBU (4 Equiv.), carbamate **2a** (1.5 Equiv.), 80 °C and 2 min residence time) (Scheme 6 and Table 5). Due to the close chemical reactivities of carbamates **2b** and **2c** and sulfonamides **3a** and **3b,** both chlorpropamide **1b** and tolbutamide **1c** were prepared in the same conversions at comparable reaction conditions (Table 5 and Figure 3).

**Table 5: The investigation of residence time and temperature in the reaction towards chlorpropamide 1b and tolbutamide 1c from carbamate 2b and 2c^{a}**

| Entry | Res. Time (min) | Temp. (°C) | Conversion (%)^{d} |
|---|---|---|---|
| 1 | 2 | 80 | 100 |
| 2 | 2 | 50 | 76 |
| 3 | 5 | 50 | 81 |
| 4 (b) | 2 | 80 | 100 |
| 5 (c) | 2 | 80 | 100 |

| | | | |
|---|---|---|---|
| Standard conditions: ^{a}Sulfonamide **3b** and **3c** (1 M, 1 Equiv.) premixed with DBU (4 Equiv.), carbamate **2b** and **2c** (1.5 Equiv.), ^{b}Sulfonamide **3a** and **3b** (1 M, 1 Equiv.) premixed with DBU (3 Equiv.), carbamate **2b** and **2c** (1.5 Equiv.), ^{c}Sulfonamide **3a** and **3b** (2 M, 1 Equiv.) premixed with DBU (3 Equiv.), carbamate **2b** and **2c** (1.5 Equiv.), ^{d}Conversion determined by HPLC. | | | |

The treatment of sulfonamide **3b** and **3c** (1 M, 1 Equiv.) (4 Equiv.) with carbamate **2b** and **2c** (1.5 Equiv.), in the presence of DBU (4 Equiv.) at 80 °C and 2 min residence time afforded both chlorpropamide **1b** and tolbutamide **1c** in full conversion (Table 5, Entry 1). A decrease in temperature was accompanied by a decrease in conversion (Table 5, Entry 2). Increase in residence time slightly increased conversion (Table 5, Entry 3). Full conversion was still achieved with reduced DBU (3 Equiv.) (Table 5, Entry 4). Reaction concentration was successfully increased to 2M affording chlorpropamide **1b** and tolbutamide **1c** in full conversion (Table 5, Entry 5). These observations lead to more comprehensive investigations using 2 M reagents at 80 °C (see Figure 3).

From Figure 3 it can be seen that, conversion generally improved with increase in both DBU and carbamates **2b** and **2c** equivalents. The use of carbamates **2b** and **2c** (1 Equiv.) resulted in poor conversion. The optimum conditions, based on the flow synthesis setup utilised in this embodiment of the invention, were found to be sulfonamide (2 M, 1 Equiv.), DBU (3 Equiv.), carbamate **2b** and **2c** (1.5 Equiv.), 80 °C and 0.5 min residence time to afford both chlorpropamide **1b** and tolbutamide **1c** in full conversion.

The flow synthesis preparation of chlorpropamide **1b** and tolbutamide **1c** was scaled up in a Chemtrix Kiloflow^{®} continuous flow system (18.4 ml total volume). A conversion of 98 % and an isolated yield of 97 % where achieved for both compounds, with a throughput of 592 g/h and 579 g/h for chlorpropamide **1b** and tolbutamide **1c** respectively.

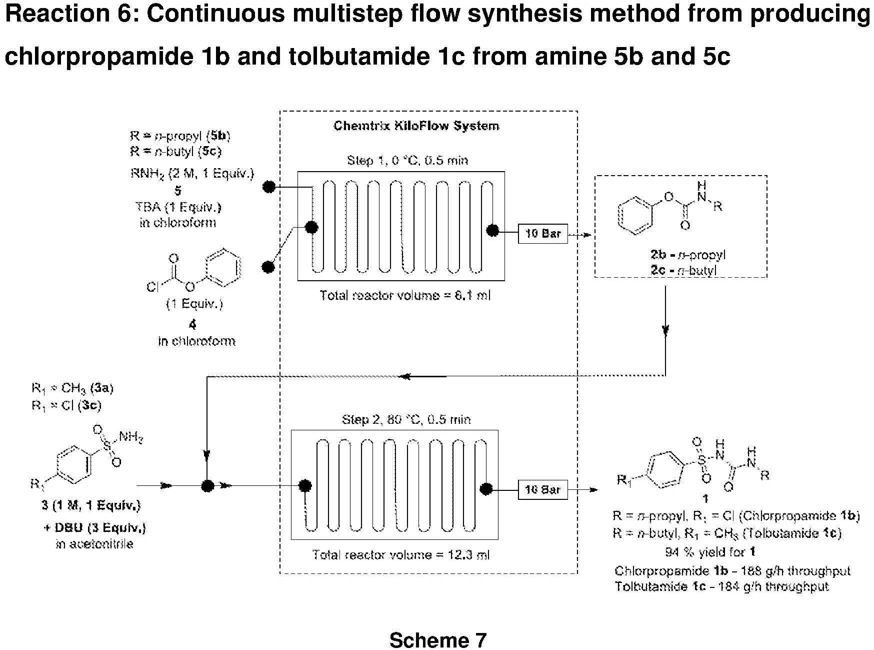

Based on the learnings from the experiments detailed above in respect of the single step reactions, the inventors successfully performed a multistep synthesis of chlorpropamide **1b** and tolbutamide **1c** from amine **5b** and **5c** via the carbamate **2b** and **2c** intermediates. This process was performed in a Chemtrix Kiloflow^{®} flow system (18.4 ml total reactor volume) to afford chlorpropamide **1b** and tolbutamide **1c** in 95 % conversion and 94 % isolated yield with a throughput of 188 g/h and 184 g/h respectively (Scheme 7). It will be appreciated that the reagents and the reaction conditions shown in Scheme 7, including the use of free amines **5b** and **5c,** can be altered according to the teachings in the experiments above and other substitutions which the skilled person would consider to have a high likelihood of success.

The inventors have developed rapid and highly efficient flow synthesis methodologies for the preparation of the sulfonylurea compounds gliclazide, chlorpropamide and tolbutamide. The compounds were efficiently prepared throughputs of 82-592 g/h (91-97 % yield) by single step synthesis. Furthermore, the compounds were efficiently prepared at multigram scale using continuous flow technology with a multistep synthesis at a throughput of 26-188 g/h and yields between 87-94 % inside 4 minutes total residence time. In addition, it is particularly significant that the important carbamate intermediates for these compounds were prepared in very high throughputs of 330-556 g/h (93-98 % yield).

### Spectroscopic data

### Carbamate 2a

Following procedure **1,** carbamate **2a** was afforded as a white solid, mp 139-140 °C; ¹H NMR (400 MHz, CDCl3) δ 7.26 (t, *J* = 7.8 Hz, 2H), 7.15 - 6.98 (m, 3H), 5.93 (s, 1H), 3.14 (t, *J* = 7.7 Hz, 2H), 2.54 (dd, *J* = 8.3, 4.6 Hz, 2H), 2.41 (t, J= 6.8 Hz, 2H), 1.57 (dt, J= 15.3, 5.5 Hz, 3H), 1.51 - 1.30 (m, 3H). ¹³C NMR (101 MHz, CDCl3) δ 150.8, 129.3, 125.2, 121.5, 115.4, 62.4, 40.3, 32.3, 25.5.

### Carbamate 2b

Following procedure **1,** carbamate **2b** was afforded as a white solid, mp 56-58 °C; ¹H NMR (400 MHz, CDCl3) δ 7.27 (t, *J* = 7.7 Hz, 2H), 7.11 (t, *J* = 7.4 Hz, 1H), 7.05 (d, *J* = 7.9 Hz, 2H), 5.01 (s, 1H), 3.15 (q, *J* = 6.7 Hz, 2H), 1.51 (h, *J* = 7.4 Hz, 2H), 0.88 (t, *J* = 7.4 Hz, 3H). ¹³C NMR (101 MHz, CDCl3) δ 153.6, 150.1, 128.2, 124.2, 120.6, 41.9, 22.0, 10.2.

### Carbamate 2c

Following procedure **1,** carbamate **2c** was afforded as a white solid, mp 38-40 °C; ¹H NMR (400 MHz, DMSO-*d*6) δ 7.72 (t, *J* = 5.7 Hz, 1H), 7.37 (t, *J* = 7.7 Hz, 2H), 7.19 (t, *J* = 7.4 Hz, 1H), 7.09 (d, *J* = 7.9 Hz, 2H), 3.07 (q, *J* = 6.6 Hz, 2H), 1.46 (p, *J* = 7.1 Hz, 2H), 1.33 (h, *J* = 7.3 Hz, 2H), 0.90 (t, *J* = 7.3 Hz, 3H). 13C NMR (101 MHz, DMSO-d6) δ 154.8, 151.6, 129.7, 125.3, 122.4, 31.8, 19.9, 14.1.

### Gliclazide 1a

Following procedure **1**, gliclazide **1a** was afforded as a white solid, mp 165-169 °C; ¹H NMR (400 MHz, CDCl3) δ 8.70 (d, *J* = 65.3 Hz, 1H), 7.88 (t, J= 6.8 Hz, 2H), 7.24 (t, *J* = 6.6 Hz, 2H), 6.23 - 5.77 (m, 1H), 3.22 (s, 1H), 2.75 (s, 1H), 2.62 - 2.39 (m, 3H), 2.35 (s, 3H), 1.87 (d, *J* = 36.4 Hz, 2H), 1.37 (s, 5H). ¹³C NMR (101 MHz, CDCl3) δ 151.1, 144.4, 135.3, 128.4, 127.3, 64.3, 39.3, 33.4, 29.9, 20.6.

### Chlorpropamide 1b

Following procedure **1,** chlorpropamide **1b** was afforded as a white solid, mp 128-130 °C; ¹H NMR (400 MHz, DMSO-*d*6) δ 10.66 (s, 1H), 7.91 (d, 2H), 7.69 (d, 2H), 6.52 (d, *J* = 5.9 Hz, 1H), 2.91 (q, *J* = 6.7 Hz, 2H), 1.34 (h, *J* = 7.4 Hz, 2H), 0.76 (t, *J* = 7.4 Hz, 3H). ¹³C NMR (101 MHz, DMSO) δ 151.8, 139.7, 138.5, 129.7, 129.6, 41.5, 22.9, 11.8.

### Tolbutamide 1c

Following procedure **1,** tolbutamide **1c** was afforded as a white solid, mp 128-130 °C; ¹H NMR (400 MHz, DMSO-*d*6) δ 10.45 (s, 1H), 7.78 (d, 2H), 7.41 (d, *J* = 7.9 Hz, 2H), 6.42 (t, *J* = 5.8 Hz, 1H), 2.94 (q, *J* = 6.6 Hz, 2H), 2.39 (s, 3H), 1.30 (p, *J* = 7.1 Hz, 2H), 1.17 (h, *J* = 7.4 Hz, 2H), 0.82 (t, *J* = 7.3 Hz, 3H). ¹³C NMR (101 MHz, DMSO) δ 151.7, 144.0, 137.9, 129.9, 127.7, 31.7, 21.5, 19.8, 14.0.

This above description of some of the illustrative embodiments of the invention is to indicate how the invention can be made and carried out. Those of ordinary skill in the art will know that various details may be modified thereby arriving at further embodiments, but that many of these embodiments will remain within the scope of the invention.

## Claims

1. A flow synthesis process for producing a sulfonylurea compound of the Formula **1** or its pharmaceutically acceptable salts, 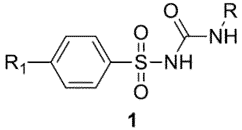 the process comprising the steps of:
a) preparing the carbamate of Formula **2** 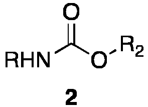 by reacting free amine RNH₂ or amine HCl salt with haloformate of Formula **4** in the presence of a base selected from 1,8-Diazabicyclo[5.4.0]undec-7-ene (DBU), trimethylamine (TEA), and tributyl amine (TBA), wherein the free amine RNH₂ or amine HCl salt to base molar ratio is about 1:1.5 to about 1:3, 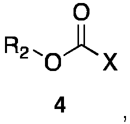
b) reacting the carbamate of Formula **2** with the sulfonamide of Formula **3** in the presence of a base selected from 1,8-Diazabicyclo[5.4.0]undec-7-ene (DBU), trimethylamine (TEA), and tributyl amine (TBA), 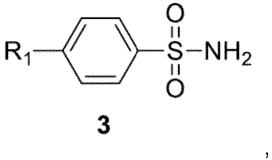 wherein
R₂ is selected from aryl and alkyl,
R₁ is selected from Cl and -CH₃,
R is selected from propyl, butyl, and 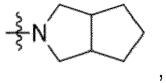 and
X is selected from CI, Br, and I.

2. The process according to claim 1, wherein the process is a continuous multi-step process without the isolation of any intermediates.

3. The process according to claim 1 or claim 2, wherein the reaction of step (a) is performed using aryl chloroformate, for example phenyl chloroformate.

4. The process according to any one of the preceding claims, wherein the reaction of step (a) is performed in an organic solvent selected from the group consisting of chloroform, dichloromethane, acetonitrile, and mixtures thereof.

5. The process according to claim 4, wherein the reaction of step (a) is performed in chloroform.

6. The process according to any one of the preceding claims, wherein the base used in step (a) and step (b) is the same.

7. The process according to any one of the preceding claims, wherein the reaction of step (a) is thermally controlled to a temperature equal to about room temperature or less.

8. The process according to claim 7, wherein the reaction of step (a) is thermally controlled to a temperature between about 0 °C and about room temperature.

9. The process according to any one of the preceding claims, wherein the free amine RNH₂ or amine HCl salt has a concentration of about 0.1M to about 2M.

10. The process according to any one of the preceding claims, wherein the sulfonamide has a concentration of about 0.1M to about 2M.

11. The process according to any one of the preceding claims, wherein in step (b) the sulfonamide to carbamate molar ratio is about 1:1 to about 1:2.

12. The process according to claim 11, wherein in step (b) the sulfonamide to carbamate molar ratio is about 1:1.5.

13. The process according to any one of the preceding claims, wherein in step (b) the sulfonamide to base molar ratio is about 1:2 to about 1:5.

14. The process according to any one of the preceding claims, wherein the reaction of step (b) is thermally controlled to a temperature between about 70 °C and about 100 °C.

15. The process according to claim 14, wherein the reaction of step (b) is thermally controlled to a temperature of about 80 °C.

## Patentansprüche

1. Ein Fließsyntheseverfahren zur Herstellung einer Sulfonylharnstoffverbindung der Formel **1** oder ihrer pharmazeutisch verträglichen Salze, 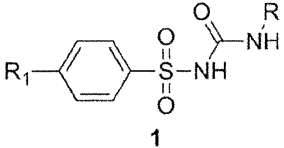 wobei das Verfahren die folgenden Schritte umfasst:
a) Herstellen des Carbamats der Formel **2** 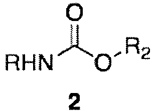 durch Umsetzung von freiem Amin RNH₂ oder Amin-HCl-Salz mit Halogenformiat der Formel **4** in Gegenwart einer Base, ausgewählt aus 1,8-Diazabicyclo[5.4.0]undec-7-en (DBU), Trimethylamin (TEA) und Tributylamin (TBA), wobei das Molverhältnis von freiem Amin RNH₂ oder Amin-HCl-Salz zu Base etwa 1:1,5 bis etwa 1:3 beträgt, 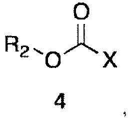
b) Umsetzen des Carbamats der Formel **2** mit dem Sulfonamid der Formel 3 in Gegenwart einer Base, ausgewählt aus 1,8-Diazabicyclo[5.4.0]undec-7-en (DBU), Trimethylamin (TEA) und Tributylamin (TBA), 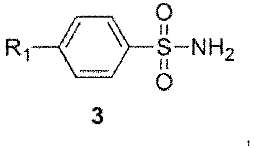
wobei R₂ ausgewählt ist aus Aryl und Alkyl,
R₁ ausgewählt ist aus CI und -CH₃,
R ausgewählt ist aus Propyl, Butyl und 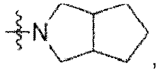 und
X ausgewählt ist aus Cl, Br und I.

2. Verfahren nach Anspruch 1, wobei es sich um ein kontinuierliches mehrstufiges Verfahren ohne Isolierung von Zwischenprodukten handelt.

3. Verfahren nach Anspruch 1 oder Anspruch 2, wobei die Reaktion in Schritt (a) unter Verwendung von Arylchlorformiat, beispielsweise Phenylchlorformiat, durchgeführt wird.

4. Verfahren nach einem der vorhergehenden Ansprüche, wobei die Reaktion von Schritt (a) in einem organischen Lösungsmittel durchgeführt wird, das aus der Gruppe ausgewählt ist, die aus Chloroform, Dichlormethan, Acetonitril und Mischungen davon besteht.

5. Verfahren nach Anspruch 4, wobei die Reaktion von Schritt (a) in Chloroform durchgeführt wird.

6. Verfahren nach einem der vorhergehenden Ansprüche, wobei die in Schritt (a) und Schritt (b) verwendete Base dieselbe ist.

7. Verfahren nach einem der vorhergehenden Ansprüche, wobei die Reaktion von Schritt (a) thermisch auf eine Temperatur von etwa Raumtemperatur oder weniger geregelt wird.

8. Verfahren nach Anspruch 7, wobei die Reaktion von Schritt (a) thermisch auf eine Temperatur zwischen etwa 0 °C und etwa Raumtemperatur geregelt wird.

9. Verfahren nach einem der vorstehenden Ansprüche, wobei das freie Amin RNH₂ oder das Amin-HCl-Salz eine Konzentration von etwa 0,1 M bis etwa 2 M aufweist.

10. Verfahren nach einem der vorhergehenden Ansprüche, wobei das Sulfonamid eine Konzentration von etwa 0,1 M bis etwa 2 M aufweist.

11. Verfahren nach einem der vorhergehenden Ansprüche, wobei in Schritt (b) das Molverhältnis von Sulfonamid zu Carbamat etwa 1:1 bis etwa 1:2 beträgt.

12. Verfahren nach Anspruch 11, wobei in Schritt (b) das Molverhältnis von Sulfonamid zu Carbamat etwa 1:1,5 beträgt.

13. Verfahren nach einem der vorstehenden Ansprüche, wobei in Schritt (b) das Molverhältnis von Sulfonamid zu Base etwa 1:2 bis etwa 1:5 beträgt.

14. Verfahren nach einem der vorstehenden Ansprüche, wobei die Reaktion in Schritt (b) thermisch auf eine Temperatur zwischen etwa 70 °C und etwa 100 °C geregelt wird.

15. Verfahren nach Anspruch 14, wobei die Reaktion in Schritt (b) thermisch auf eine Temperatur von etwa 80 °C geregelt wird.

## Revendications

1. Procédé de synthèse en flux pour produire un composé de sulfonylurée de formule 1 ou ses sels pharmaceutiquement acceptables, 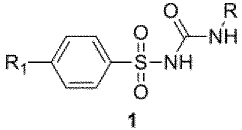 le procédé comprenant les étapes de :
a) préparation du carbamate de formule 2 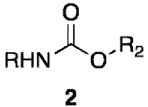 en faisant réagir l'amine libre RNH₂ ou le sel HCl d'amine avec l'haloformiate de formule 4 en présence d'une base choisie parmi le 1,8-diazabicyclo[5.4.0]undéc-7-ène (DBU), la triméthylamine (TEA) et la tributylamine (TBA), le rapport molaire de l'amine libre RNH₂ ou du sel HCl d'amine à la base étant d'environ 1:1,5 à environ 1:3, 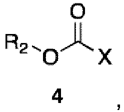
b) réaction du carbamate de formule 2 avec le sulfonamide de formule 3 en présence d'une base choisie parmi le 1,8-diazabicyclo[5.4.0]undéc-7-ène (DBU), la triméthylamine (TEA) et la tributylamine (TBA), 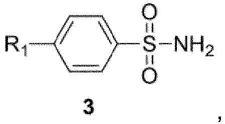 où
R₂ est choisi parmi des groupes aryle et alkyle,
R ₁ est choisi parmi CI et -CH₃,
R est choisi parmi des groupes propyle, butyle et 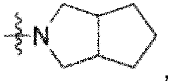 et
X est choisi parmi CI, Br et I.

2. Procédé selon la revendication 1, le procédé étant un procédé continu en plusieurs étapes sans isolement d'aucun intermédiaire.

3. Procédé selon la revendication 1 ou la revendication 2, dans lequel la réaction de l'étape (a) est réalisée en utilisant un chloroformiate d'aryle, par exemple le chloroformiate de phényle.

4. Procédé selon l'une quelconque des revendications précédentes, dans lequel la réaction de l'étape (a) est réalisée dans un solvant organique choisi dans le groupe consistant en chloroforme, dichlorométhane, acétonitrile et leurs mélanges.

5. Procédé selon la revendication 4, dans lequel la réaction de l'étape (a) est réalisée dans le choroforme.

6. Procédé selon l'une quelconque des revendications précédentes, dans lequel la base utilisée dans l'étape (a) et l'étape (b) est la même.

7. Procédé selon l'une quelconque des revendications précédentes, dans lequel la réaction de l'étape (a) est contrôlée thermiquement à une température égale ou inférieure à environ la température ambiante.

8. Procédé selon la revendication 7, dans lequel la réaction de l'étape (a) est contrôlée thermiquement à une température entre environ 0°C et environ la température ambiante.

9. Procédé selon l'une quelconque des revendications précédentes, dans lequel l'amine libre RNH₂ ou le sel HCl d'amine a une concentration d'environ 0,1 M à environ 2 M.

10. Procédé selon l'une quelconque des revendications précédentes, dans lequel le sulfonamide a une concentration d'environ 0,1 M à environ 2 M.

11. Procédé selon l'une quelconque des revendications précédentes, dans lequel, dans étape (b), le rapport molaire du sulfonamide au carbamate est d'environ 1:1 à environ 1:2.

12. Procédé selon l'une quelconque des revendications précédentes, dans lequel, dans étape (b), le rapport molaire du sulfonamide au carbamate est d'environ 1:1,5.

13. Procédé selon l'une quelconque des revendications précédentes, dans lequel, dans étape (b), le rapport molaire du sulfonamide à la base est d'environ 1:2 à environ 1:5.

14. Procédé selon l'une quelconque des revendications précédentes, dans lequel la réaction de l'étape (b) est contrôlée thermiquement à une température entre environ 70°C et environ 100°C.

15. Procédé selon la revendication 14, dans lequel la réaction de l'étape (b) est contrôlée thermiquement à une température d'environ 80°C.
